Europäisches Patentamt

(19) European Patent Office

Office européen des brevets

(11) Publication number: **0 232 573 B1**

# (12) EUROPEAN PATENT SPECIFICATION

(45) Date of publication of patent specification:
06.02.91 Bulletin 91/06

(51) Int. Cl.⁵: **G01N 3/20**

(21) Application number: **86300893.4**

(22) Date of filing: **10.02.86**

(54) **Method and apparatus for the non-destructive testing of reconstituted wood panels.**

(43) Date of publication of application:
19.08.87 Bulletin 87/34

(45) Publication of the grant of the patent:
06.02.91 Bulletin 91/06

(84) Designated Contracting States:
GB SE

(56) References cited:
GB-A- 1 018 726
GB-A- 2 043 265
GB-A- 2 148 517
US-A- 4 213 349
US-A- 4 313 348

(73) Proprietor: Her Majesty the Queen in Right of
the Province of Alberta as represented by the
Minister of Energy and Natural Resources
The Office of the Deputy Minister Energy
Resources 9915-108 Street
Edmonton Alberta T5K 2C9 (CA)

(72) Inventor: Porter, Andrew W.
7531 - Schaefer Avenue
Richmond British Columbia, V6Y 2W7 (CA)
Inventor: Bach, Lars
7912 - 147 Street
Edmonton Alta, T5R 0X7 (CA)

(74) Representative: Stanley, David William et al
APPLEYARD LEES & CO. 15 Clare Road
Halifax
West Yorkshire HX1 2HY (GB)

## Description

This invention relates to the non-destructive testing of a reconstituted wood panel, such as a sheet of plywood.

Wood panels, generically termed "reconstituted wood panels", include such fabricated products as plywood, flakeboard, hardboard, particleboard, waferboard, oriented strand board and the like. Such panels are manufactured in the form of large relatively thin sheets. Typically, such a panel might be about 1.2 metres in width and 2.4 metres in length.

As a consequence of the process of manufacture, such panels display a greater propensity to warpage and sagging than a natural wood product.

It is desirable to be able to establish, at a relatively high speed and in a non-destructive fashion, values for the panel which can be used to calculate a measure of the stiffness of the panel. We have found that the stiffness of a panel correlates with the modulus of elasticity (MOE), and, as a result, with the modulus of rupture (MOR) of the panel.

At this time, to our knowledge, there is no machine available which can, in a matter of seconds, grade a panel by yielding such values. There is, in common use in the industry, a machine known at the Post Flexure machine. In the use of this machine, the panel is threaded on edge into two spaced vertical frames, which frames are then rotated in opposite directions to flex the central part of the panel. The values for the force used to effect rotation, and the extent of deflection effected are used in suitable equations to calculate a value representative of MOE. However, this machine is not suitable for grading on a production line, as it takes several minutes to test a panel with it.

U.S. Patent Number 4 313 348 discloses a method in accordance with the pre-characterizing portion of Claim 1, for stress grading timber boards, using apparatus which includes two support rollers between which a board may be positioned, load applying means arranged to displace transversely the board and a load transducer for measuring the load applied by the load applying means.

We evolved the following criteria for a machine that could be used to grade wood panels :

(1) it must accommodate large panels ;

(2) it must distribute the deflecting load substantially uniformly and linearly across the full width of the panel, in spite of the warping or sagging nature of said panel ;

(3) it must cope with the fact that the load deflection (or moment vs. deflection) curve for a wood panel is not, typically, linear in the proximity of the zero point ; and

(4) it must be relatively fast in operation and capable of being upgraded to production line speeds.

Preferred embodiments of the invention aim to meet these criteria.

According to a first aspect of the invention, there is provided a method of testing the stiffness of a wood panel which has a substantially linear relationship between load and deflection when tested to develop a load deflection curve, the method comprising the steps of :

contacting one major surface of the panel substantially across its width with a loading member and biasing said member to apply a substantially linear and uniform first load to the panel whilst supporting the other major surface of the panel along two spaced lines bracketing and parallel to a line of application of said first load, thereby to deflect the panel, said load being of a magnitude to fall on the substantially linear portion of the load deflection curve for the panel ;

determining the magnitude of the first load to obtain a first measurement ;

further biasing the loading member thereby to apply an incremental load to the panel in order to further deflect said panel through a second distance, said incremental load also falling on said linear portion of said load deflection curve ;

determining the magnitude of the incremental load and the extent of the deflection to obtain second and third measurements ;

using the measurements obtained to compute a measure of the stiffness of the panel ;

characterised in that said wood panel is a reconstituted wood panel and apparatus comprising a double-acting cylinder connected at one end with a support and at the other end with said loading member is provided, whereby extension of one end of the cylinder applies said first load and extension of the other end of the cylinder applies said incremental load.

Preferably, said further biasing occurs in quick succession to the application of the first load. Preferably, said first load is predetermined. Said second distance may be predetermined.

The invention also provides apparatus for carrying out the method of the first aspect, the apparatus comprising :

first means for bearing against one major surface of the panel along two spaced parallel lines extending substantially across the width of the panel ;

2

second means for bearing against the other major surface of the panel along a line extending substantially across the width of the panel between the aforesaid spaced parallel line ;

third means for biasing one of said first and second means to apply a first substantially linear and uniform load across substantially the width of the panel, said first load being selected to fall on the substantially linear portion of the load deflection curve for the panel, thereby to deflect the panel across its width a first distance; and

fourth means for further biasing said one means to apply an incremental load to the panel also falling on said linear portion of said load deflection curve thereby to further deflect the panel a second distance ;

characterised in that said third and fourth means comprises a double-acting cylinder connected at one end with a support and at the other end with said one means, whereby extension of one end of the cylinder applies said first load and extension of the other end of the cylinder applies said incremental load.

The apparatus may further comprise a support frame on which said first and second means and/or said third and fourth means are mounted. Preferably, said one means is pivotally mounted for pivoting movement in a plane generally perpendicular to the main plane of a panel under test, whereby it may conform closely with the adjacent panel surface. Preferably, said one means is said second means.

Preferably, the apparatus is arranged to test a panel when substantially horizontal. Preferably, said first and second means are arranged to bear against the top and bottom surfaces respectively of a panel under test. Preferably, said first means comprises a pair of spaced rotatable members.

For a better understanding of the invention and to show how the same may be carried into effect, reference will now be made, by way of example, to the accompanying diagrammatic drawings, in which :

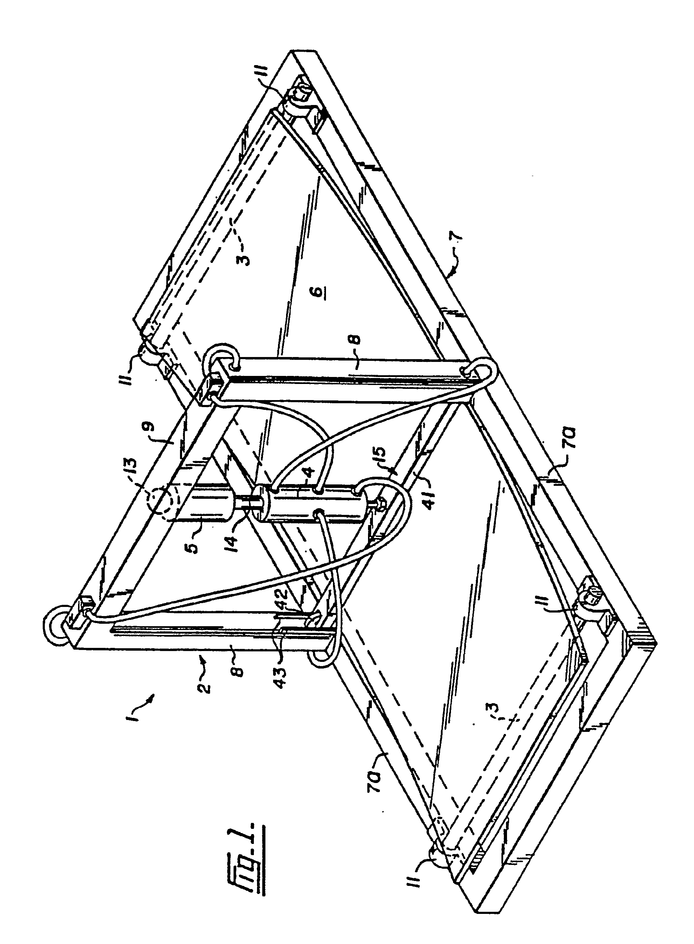

Figure 1 is a perspective view of an apparatus for the non-destructive testing of a reconstituted wood panel, showing a panel undergoing deflection ;

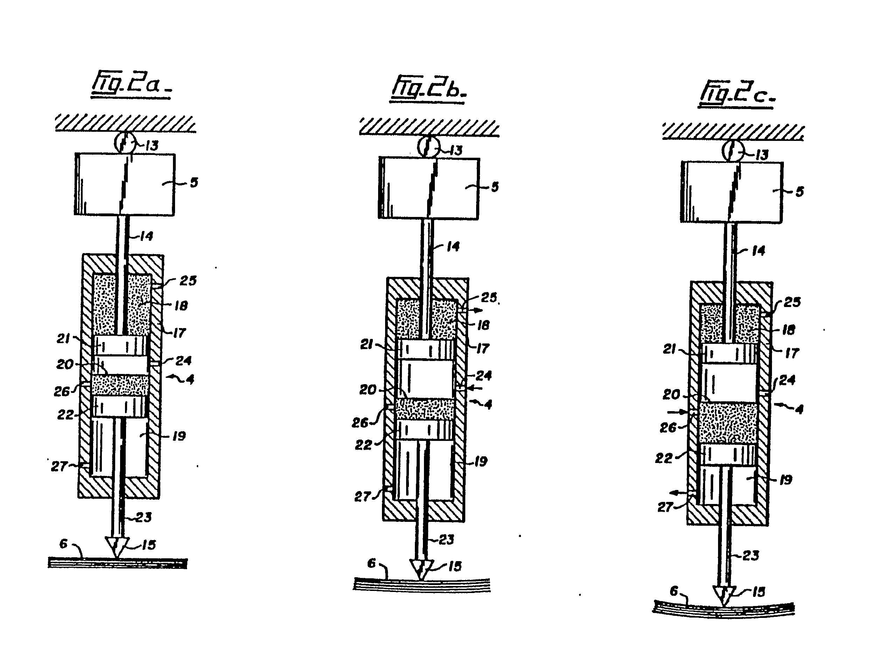

Figure 2 is a three-part sectional schematic view of a loading bar, double-acting cylinder, load cell, and universal pivot, which together make up a deflecting assembly, with the cylinder shown in a beginning mode (Figure 2a), first loading mode (Figure 2b), and second loading mode (Figure 2c) ; and

Figure 3 is a schematic view showing a pneumatic-hydraulic actuating circuit, the deflecting assembly, the panel, and a support means.

The illustrated apparatus 1 comprises, in general a frame 2, a pair of support rollers 3, a pivoted load-applying two-stage cylinder assembly 4, and a load cell 5. The rollers 3 support the wood panel 6 to be tested. The cylinder assembly 4 applies two different and sequential loadings, to pre-load and then incrementally load the panel to deflect the latter through a pre-determined distance. The load cell 5 provides a measure of the loading applied. The frame 2 carries the aforementioned components.

The support frame 2 comprises a horizontal, rectangular section 7. A pair of vertically extending posts 8 project upwardly from the side members 7a of the section 7, substantially at their mid-points. A horizontal beam 9 extends between the posts 8 and is supported thereby. The beam 9 is therefore positioned to extend transversely across the rectangular section 7, substantially at its mid-point, and in vertically spaced relation thereabove. The rectangular section 7 is of sufficient width and length so as to accommodate the wood panel 6.

The pair of parallel, spaced apart, elongate support rollers 3 are each supported by a pair of pillow blocks 11, which pillow blocks are mounted on the rectangular section side members 7a adjacent the latters' ends. The wood panel 6 may thus be supported by the rollers 3 from below and across its width, at points adjacent each of its ends.

There is thus provided means, associated with the support frame, for bearing against one major surface of the panel along two spaced, parallel lines extending substantially across the width of the panel.

The rollers 3 are utilized to provide the desired two spaced end supports for the panel, while offering no frictional resistance to panel movement which arises from central deflection thereof.

The load cell 5 is mounted to the underside of the beam 9 by a universal joint 13. The load cell 5 is connected to a piston rod 14 of the two-stage cylinder assembly 4. At its lower end, the cylinder assembly 4 includes an attached loading bar 15, which may be biased to press against the wood panel 6, to deflect the latter.

The cylinder assembly 4 comprises a cylinder body 17 which forms upper and lower chambers 18, 19 separated by a divider 20. A piston 21 is disposed in upper chamber 18 and, as previously mentioned, its piston rod 14 is connected to the load cell 5. A second piston 22 is disposed in the lower chamber 19 and its piston rod 23 is connected to the loading bar 15.

A port 24, for admitting and exhausting of pressurized air, communicates through the cylinder body wall with the bottom end of the upper chamber 18. A port 25, for admitting and exhausting of oil, communicates with the top end of the upper chamber 18.

Turning now to the lower chamber 19, a port 26, for admitting and exhausting of oil, communicates with

3

the top end of said lower chamber. And a port 27, for admitting and exhausting of pressurized air, communicates with the lower end of the lower chamber 19.

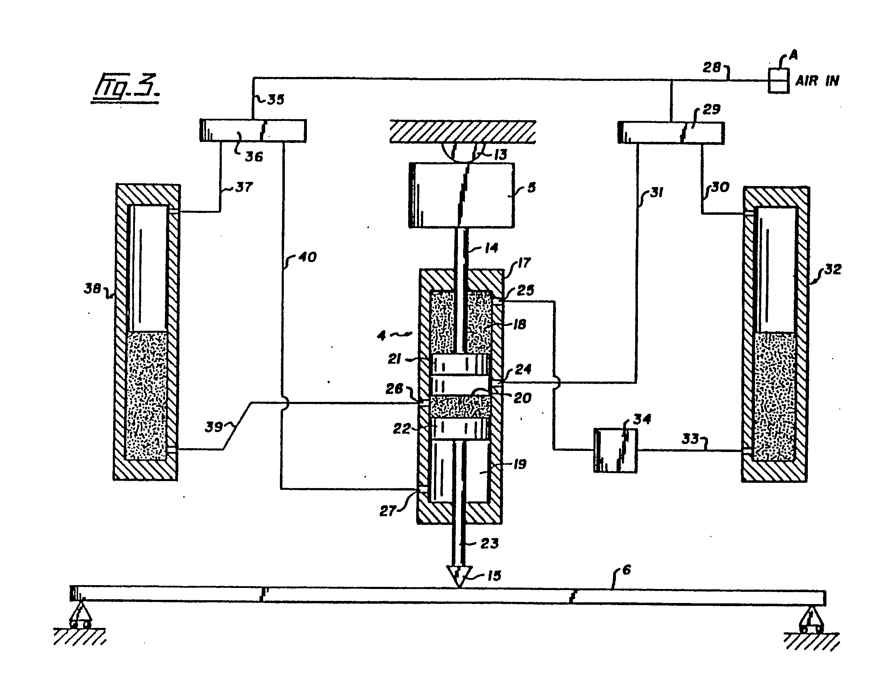

Having reference to the pneumatic-hydraulic circuit shown in Figure 3, a source A of pressurized air supplies air through line 28 to a 4-way pneumatic valve 29.

The air can move from this valve 29 through either line 30 or 31. Line 30 connects with the upper end of the pre-load oil reservoir cylinder 32. This preload reservoir cylinder 32 connects at its base with a line 33. The line 33 is controlled by a 2-way normally closed valve 34 and connects with the port 25 at the top end of the upper chamber 18. Line 31 connects with the port 24 at the lower end of the upper chamber 18.

The air source A is also connected through line 35 with 4-way pneumatic valve 36. A line 37 connects the valve 36 with the upper end of the final load reservoir cylinder 38. The lower end of cylinder 38 is connected by line 39 with the port 26 leading into the upper end of the lower chamber 19. The valve 36 is also connected by a line 40 with the port 27 leading into the lower chamber 19.

By means of this arrangement, one can first open the valve 29 and admit pressurized air from source A through lines 28 and 30 into the pre-load reservoir cylinder 32. Upon opening the valve 34, oil is forced by the air from cylinder 32 through line 33 and valve 34, to fill the upper end of the upper chamber 18. Valve 34 is then closed and the oil in chamber 18 is 'locked' in place. Valve 36 can also be opened to admit air through line 37 into the final load reservoir cylinder 38 and force oil through line 39 to the upper end of lower chamber 19. Valve 36 can then be switched to introduce air through line 40 to the lower end of chamber 19. At this point, the system is ready for use. Both pistons 22 and 21 are adjacent the divider 20 and piston rod 23 is fully retracted.

To initiate testing of a panel, the valve 29 is opened to admit air through line 31 and port 24 into the lower end of upper chamber 18. At the same time, valve 34 is opened to allow oil to be exhausted from chamber 18 into reservoir cylinder 32. As a result, the cylinder body 17 is forced downward and the piston rod 23 and loading bar 15 press downwardly against the panel. The force being applied by the loading bar 15 is monitored on the load cell 5, until it reaches a pre-determined value. This value is selected to correspond with a point that is safely on the linear portion of the moment vs deflection curve for a panel of the type being tested. At this stage, valve 34 is closed, so that the pre-load force being applied to the panel is maintained.

The valve 36 is then opened to apply air pressure through line 37 to force oil from cylinder 38 into the upper end of the lower chamber 19. This causes the piston 22 and rod 23 to move downwardly through a known travel, being the full stroke of the piston 22. When this stroke is complete, the force being exerted by the loading bar 15 is noted from the load cell 5.

The circuit is then returned to the starting condition by opening line 37 through valve 36, admitting air through valve 36 and line 40 to retract the piston 22, and opening valve 34 and admitting air through valve 29 and line 30 to depress piston 21.

By virtue of this arrangement, the following steps occur:
(1) the panel is deflected to a limited extent by applying a load of pre-determined magnitude;
(2) the panel is then further deflected through a known distance and the magnitude of the incremental load required to achieve this is measured.

From the values for the pre-load, the incremental load, the deflection arising from the incremental load and the known span and width of the panel, the stiffness of the panel can be calculated. A close approximation of the MOE can be calculated if the thickness of the panel is known.

Loading bar 15 contacts the mid-point of panel 6 from above, transmitting the loads supplied by cylinder assembly 4 to said panel. Loading bar 15 consists of an elongate rod 41 having a sheaved wheel 42 at each end thereof. The bar 15 is operative to distribute the applied loads substantially uniformly across the panel width as a line load. The loading bar 15 is threadably engaged to the lower end of lower piston rod 23 and is operative to move concomitantly therewith. Sheaved wheels 42 engage guides 43 mounted on posts 8 and function to maintain the bar 15 in longitudinal alignment. Provision of universal joint 13 ensures good contact between bar 15 and panel 6, irrespective of warping, sagging, or surface irregularities thereof.

Stated otherwise, there is provided means for bearing against the upper major surface of the panel along a line extending substantially across the width of the panel, which means are pivotally mounted on the support frame for pivoting in a plane generally perpendicular to the main plane of the panel, whereby said bearing means may conform closely with the panel surface to apply a substantially linear and uniform load across substantially the width of the panel.

EXAMPLE

This example shows the close correlation, in the calculated MOE results, which is obtained by the use

4

of the illustrated apparatus and the industry-accepted Post flexure apparatus.

Testing was conducted following the requirements of ASTM D 3043-76 (1981) Method C for the Post flexure apparatus.

The illustrated apparatus was operated in accordance with the following specific conditions :

The support rollers for plywood sample M-1-A were chosen to be spaced 1118 mm and the initial pre-determined stress level for preload was 3.5 MPa. This sample was 1219 mm wide and had a thickness of 9.44 mm. The incremental deflection following preloading was chosen to be 12.75 mm.

The materials tested were normally 1220 x 2440 x 9.5 mm panels of softwood plywood and 1220 x 2440 x 11.1 mm panels of waferboard.

The test procedure followed with respect to using both machines was as follows :

1. Each 1220 x 2440 mm sheet was cut into two 1220 x 1220 mm samples. Half were designated as A – for testing parallel to the grain direction (or along the length in the case of undirectional boards) and half were designated B – for testing across the grain direction (or along the width) ;

2. Parallel and cross dimensions were measured at mid-span ;

3. Thickness was measured at the middle of each edge and the average of the four values recorded ;

4. All of the A panels, then all of the B panels were tested on the illustrated machine ;

5. A check was made to ensure that the two load levels from the MSR machine testing (preload and final load) both fell on the straight line portion of the load deflection plot from the Post flexure machine testing ; and

6. All of the A panels and then all of the B panels were tested to failure on the Post flexure machine (per ASTM D 3043C-76).

The data derived from testing the MSR machine was used to compute MOE using the following equation :

$$MOE = \frac{L^3}{4 \cdot b \cdot d^3} \cdot \frac{\Delta P}{\Delta Y}$$

where : b = width of specimen in millimetres

d = average thickness of specimen in millimeters

L = length of span in millimetres

$\dfrac{\Delta P}{\Delta Y}$ = slope of the load deflection curve derived using two points established by the pre-load and incremental load.

The MOE from the Post flexure testing was calculated using the computations required by the aforesaid ASTM standard.

The results from the testing were as follows :

## TABLE A-1

### SUMMARY OF TESTING - MSR*AND POST FLEXURE
### * MSR designates the illustrated apparatus

| Sample Number | Thick mm | MSR MOE MPa | Post Flexure MOE MPa |
|---|---|---|---|
| M-1-A | 9.44 | 8,580 | 8,810 |
| M-2-A | 9.31 | 8,910 | 9,130 |
| M-3-A | 9.38 | 8,870 | 9,620 |
| M-4-A | 9.20 | 10,190 | 10,780 |
| M-5-A | 9.16 | 9,210 | 9,860 |
| M-6-A | 9.34 | 8,530 | 8,540 |
| M-7-A | 9.14 | 8,990 | 10,000 |
| M-8-A | 9.81 | 8,650 | 8,610 |
| M-9-A | 9.18 | 10,350 | 10,700 |
| M-10-A | 9.29 | 9,390 | 9,250 |
| M-11-A | 9.52 | 8,690 | 9,060 |
| M-12-A | 9.31 | 9,410 | 9,380 |
| M-13-A | 9.31 | 8,890 | 8,840 |
| M-14-A | 8.87 | 11,570 | 11,410 |
| M-15-A | 9.38 | 10,320 | 10,750 |
| M-16-A | 9.14 | 10,850 | 11,360 |
| M-17-A | 9.16 | 10,210 | 10,070 |
| M-18-A | 9.23 | 9,670 | 9,630 |
| M-19-A | 9.16 | 10,720 | 10,530 |
| M-20-A | 9.13 | 10,380 | 10,400 |
| Average | 9.27 | 9,620 | 9,840 |

Test Material - 9.5 mm Spruce Plywood
Test Direction - Machine or Parallel Direction
Conditioning - As Received
Predetermined Initial Stress Level - 3.5 MPa
Stroke of Final Load Cylinder - 12.75 mm
Test Span - 1118 mm

## TABLE A-2

### SUMMARY OF TESTING - MSR AND POST FLEXURE

| Sample Number | Thick mm | MSR MOE MPa | Post Flexure MOE MPa |
|---|---|---|---|
| T-1-B | 9.54 | 700 | 740 |
| T-2-B | 9.39 | 540 | 520 |
| T-3-B | 9.50 | 790 | 800 |
| T-4-B | 9.26 | 590 | 640 |
| T-5-B | 9.18 | 580 | 620 |
| T-6-B | 9.54 | 610 | 580 |
| T-7-B | 9.40 | 780 | 720 |
| T-8-B | 9.84 | 540 | 560 |
| T-9-B | 9.12 | 700 | 710 |
| T-10-B | 9.28 | 620 | 670 |
| T-11-B | 9.59 | 710 | 760 |
| T-12-B | 9.30 | 660 | 730 |
| T-13-B | 9.42 | 920 | 1,060 |
| T-14-B | 9.07 | 560 | 700 |
| T-15-B | 9.41 | 620 | 740 |
| T-16-B | 9.33 | 580 | --- |
| T-17-B | 9.09 | 660 | 850 |
| T-18-B | 9.02 | 550 | --- |
| T-19-B | 9.14 | 650 | 790 |
| T-20-B | 8.98 | 580 | 760 |
| Average | 9.32 | 650 | 720 |

Test Material - 9.5 mm Spruce Plywood
Test Direction - Transverse Direction
Conditioning - As Received
Predeterming Initial Stress Level - 0.8 MPa
Stroke of Final Load Cylinder - 12.75 mm
Test Span - 1118 mm

## TABLE A-3

### SUMMARY OF TESTING – MSR AND POST FLEXURE

| Sample Number | Thick mm | MSR MOE MPa | Post Flexure MOE MPa |
|---|---|---|---|
| U1A | 11.09 | 4,360 | 4,260 |
| U2A | 11.20 | 4,460 | 4,630 |
| U3A | 11.28 | 4,280 | 4,430 |
| U4A | 10.44 | 3,920 | 3,890 |
| U5A | 12.17 | 3,860 | 4,040 |
| U6A | 11.45 | 4,320 | 4,370 |
| U7A | 10.86 | 4,040 | 4,020 |
| U8A | 11.76 | 4,420 | 4,530 |
| U9A | 11.99 | 4,380 | 4,520 |
| U10A | 11.57 | 4,710 | 5,020 |
| U11A | 11.71 | 4,470 | 4,690 |
| U12A | 11.30 | 4,760 | 5,140 |
| U13A | 11.90 | 4,310 | 4,560 |
| U14A | 11.19 | 4,930 | 5,390 |
| U15A | 11.83 | 4,320 | 4,480 |
| U16A | 11.47 | 4,570 | 4,700 |
| U17A | 11.22 | 4,850 | 4,890 |
| U18A | 12.09 | 3,880 | 3,820 |
| U19A | 11.21 | 5,150 | 5,310 |
| U20A | 12.00 | 4,210 | 4,170 |
| Average | 11.49 | 4,410 | 4,540 |

Test Run - Waferboard
Nominal Thickness - 11.1 mm
Test Direction - Parallel to Machine Direction
Predetermined Initial Stress Level - 2.758 MPa
Stroke of Final Cylinder - 12.75 mm
Test Span - 1118 mm

## TABLE A-4

### SUMMARY OF TESTING - MSR AND POST FLEXURE

| Sample Number | Thick mm | MSR MOE MPa | Post Flexure MOE MPa |
|---|---|---|---|
| U1B | 11.13 | 3,920 | 3,980 |
| U2B | 10.82 | 3,750 | 3,930 |
| U3B | 11.34 | 4,300 | 4,470 |
| U4B | 10.74 | 4,070 | 4,360 |
| U5B | 11.50 | 4,020 | 4,390 |
| U6B | 11.81 | 3,700 | 3,870 |
| U7B | 10.90 | 4,360 | 4,430 |
| U8B | 12.02 | 3,680 | 3,900 |
| U9B | 11.49 | 4,150 | 4,500 |
| U10B | 11.62 | 3,900 | 4,160 |
| U11B | 11.61 | 3,900 | 4,090 |
| U12B | 11.56 | 3,780 | 3,830 |
| U13B | 11.67 | 4,380 | 4,700 |
| U14B | 11.36 | 4,610 | 4,820 |
| U15B | 11.68 | 4,270 | 4,650 |
| U16B | 11.90 | 3,940 | 4,240 |
| U17B | 11.69 | 4,300 | 4,570 |
| U18B | 11.26 | 4,680 | 4,950 |
| U19B | 11.43 | 4,480 | 4,640 |
| U20B | 11.27 | 4,660 | 5,020 |
| Average | 11.44 | 4,140 | 4,380 |

Test Run - Waferboard
Nominal Thickness - 11.1 mm
Test Direction - Waferboard Across Machine Direction
Predetermined Initial Stress Level - 2.758 MPa
Stroke of Final Cylinder - 12.75 mm
Test Span - 1118 mm

## Claims

1. A method of testing the stiffness of a wood panel (6) which has a substantially linear relationship between load and deflection when tested to develop a load deflection curve, the method comprising the steps of :

contacting one major surface of the panel (6) substantially across its width with a loading member (15) and biasing said member (15) to apply a substantially linear and uniform first load to the panel (6) whilst supporting the other major surface of the panel along two spaced lines (3) bracketing and parallel to a line of application of said first load, thereby to deflect the panel (6), said load being of a magnitude to fall on the substantially linear portion of the load deflection curve for the panel (6) ;

determining the magnitude of the first load to obtain a first measurement ;

further biasing the loading member (15) thereby to apply an incremental load to the panel (6) in order to further deflect said panel (6) through a second distance said incremental load also falling on said linear portion of said load deflection curve ;

determining the magnitude of the incremental load and the extent of the deflection to obtain second and third measurements ;

using the measurements obtained to compute a measure of the stiffness of the panel (6) ;

characterised in that said wood panel (6) is a reconstituted wood panel and apparatus (1) comprising a double-acting cylinder (4) connected at one end with a support (9) and at the other end with said loading member (15) is provided, whereby extension of one end of the cylinder (4) applies said first load and extension of the other end of the cylinder (4) applies said incremental load.

2. A method according to Claim 1, wherein said further biasing occurs in quick succession to the application of the first load.

3. A method according to Claim 1 or Claim 2, wherein said first load is predetermined.

4. A method according to any of the preceding Claims, wherein said second distance is predetermined.

5. Apparatus for carrying out the method of Claims 1 to 4, the apparatus comprising :

first means (3) for bearing against one major surface of the panel (6) along two spaced parallel lines extending substantially across the width of the panel

second means (15) for bearing against the other major surface of the panel (6) along a line extending substantially across the width of the panel (6) between the aforesaid spaced parallel line ;

third means (4) for biasing one of said first and second means (3, 15) to apply a first substantially linear and uniform load across substantially the width of the panel (6), said first load being selected to fall on the substantially linear portion of the load deflection curve for the panel (6), thereby to deflect the panel across its width a first distance ; and

fourth means (4) for further biasing said one means (3, 15) to apply an incremental load to the panel (6) also falling on said linear portion of said load deflection curve thereby to further deflect the panel (6) a second distance ;

characterised in that said third and fourth means (4) comprises a double-acting cylinder (4) connected at one end with a support (9) and at the other end with said one means (3, 15), whereby extension of one end of the cylinder (4) applies said first load and extension of the other end of the cylinder (4) applies said incremental load.

6. Apparatus according to Claim 5, further comprising a support frame on which said first and second means and/or said third and fourth means are mounted.

7. Apparatus according to Claim 5 or Claim 6, wherein said one means is pivotally mounted for pivoting movement in a plane generally perpendicular to the main plane of a panel under test, whereby it may conform closely with the adjacent panel surface.

8. Apparatus according to any of Claims 5 to 7, wherein said one means is said second means.

9. Apparatus according to any of Claims 5 to 8, the apparatus being arranged to test a panel when substantially horizontal.

10. Apparatus according to Claim 9, wherein said first and second means are arranged to bear against the top and bottom surfaces respectively of a panel under test.

11. Apparatus according to any of Claims 5 to 10, wherein said first means comprises a pair of spaced rotatable members.


## Ansprüche

1. Verfahren zum Prüfen der Steifigkeit einer Holztafel (6), die beim Prüfen ein im wesentlichen lineares Verhältnis zwischen Belastung und Durchbiegung aufweist, um eine Belastungs-Durchbiegungs-Kurve zu ergeben, mit den folgenden Verfahrensschritten :

Es wird eine größere Fläche der Tafel (6) im wesentlichen quer über ihre Breite mit einem Belastungselement in Berührung gebracht und dieses Element (15) derart beaufschlagt, daß es eine im wesentlichen linear und gleichförmige erste Last auf die Tafel (6) aufbringt, während die andere größere Fläche der Tafel entlang zweier einen gegenseitigen Abstand aufweisender Linien (3) unterstützt wird, die parallel zu einer Linie der Aufbringung der ersten Last verlaufen, so daß sich die Tafel (6) durchbiegt, wobei die Last eine solche Größe aufweist, daß sie in den im wesentlichen linearen Bereich der Belastungs-Durchbiegungs-Kurve der Tafel (6) fällt ;

es wird die Größe der ersten Last bestimmt, um eine erste Messung zu erhalten ;

das Belastungselement (15) wird weiterhin beaufschlagt, um damit eine inkrementelle Last auf die Tafel (6) aufzubringen und damit die Tafel (6) um einen zweiten Abstand weiterhin durchzubiegen, wobei die inkrementelle Last ebenfalls in den genannten linearen Bereich der Belastungs-Durchbiegungs-Kurve fällt;

es werden die Größe der inkrementellen Last und das Maß der Durchbiegung bestimmt, um die zweite und dritte Messung zu erhalten ;

es werden die erhaltenen Messungen ausgenutzt, um ein Maß der Steifigkeit der Tafel (6) zu erhalten;

dadurch gekennzeichnet, daß die Holztafel (6) eine zusammengesetzte Holztafel (Sperrholz, Holspanplatte usw.) ist, und daß ein Gerät (1) vorgesehen ist, umfassend einen doppeltwirkenden Zylinder (4), dessen eines Ende mit einem Träger (9), und dessen anderes Ende mit dem Belastungselement (15) zusammenarbeitet, wobei das Ausfahren des einen Endes des Zylinders (4) eine erste Last aufbringt, und das Ausfahren des anderen Endes des Zylinders (4) die inkrementelle Last aufbringt.

2. Verfahren nach Anspruch 1, dadurch gekennzeichnet, daß eine weitere Beaufschlagung in schneller Folge auf die Aufbringung der ersten Last folgt.

3. Verfahren nach Anspruch 1 oder 2, dadurch gekennzeichnet, daß die erste Last vorgegeben ist.

4. Verfahren nach einem der vorausgegangenen Ansprüche, dadurch gekennzeichnet, daß der zweite Abstand vorgegeben ist.

5. Gerät zum Durchführen des Verfahrens nach einem der Ansprüche 1 bis 4, umfassend :

ein erstes Mittel (3), das sich an die eine größere Fläche der Tafel (6) entlang zweier einen gegenseitigen Abstand aufweisenden paralleler Linien anlegt, die sich im wesentlichen über die Breite der Tafel erstrecken ;

ein zweites Mittel (15) das sich gegen die andere größere Fläche der Tafel (6) entlang einer Linie anlegt, die sich im wesentlichen über die Breite der Tafel (6) zwischen den beiden zuvor genannten, einen gegenseitigen Abstand aufweisenden parellelen Linien anlegt ;

ein drittes Mittel (4) zum Beaufschlagen eines der beiden genannten ersten oder zweiten Mittel (3, 15) um eine erste, im wesentlichen lineare und gleichförmige Last über im wesentlichen die gesamte Breite der Tafel (6) aufzubringen, wobei die erste Last derart gewählt ist, daß sie in den im wesentlichen linearen Bereich der Belastungs-Durchbiegungs-Kurve der Tafel (6) fällt, und die Tafel (6) über ihre Breite um einen ersten Abstand verbiegt ; und

ein viertes Mittel (4) zum weiteren Beaufschlagen der beiden ersten Mittel (3, 15) zum Aufbringen einer inkrementellen Last auf die Tafel (6), die ebenfalls in den linearen Bereich der Belastungs-Durchbiegungs-Kurve fällt, um die Tafel (6) um einen zweiten Abstand weiterhin durchzubiegen ;

dadurch gekennzeichnet, daß das dritte und vierte Mittel (4) einen doppeltwirkenden Zylinder (4) umfassen, der mit seinem einen Ende mit einem Träger (9), und mit seinem anderen Ende mit einem der beiden ersten Mittel (3, 15) verbunden ist, wobei das Ausfahren des einen Endes des Zylinders (4) die genannte erste Last, und das Ausfahren des anderen Endes des Zylinders (4) die genannte inkrementelle Last aufbringen.

6. Gerät der der Anspruch 5, dadurch gekennzeichnet, daß ein Tragrahmen vorgesehen ist, auf welchem das erste und das zweite Mittel und/oder das dritte und das vierte Mittel montiert sind.

7. Gerät nach einem der Ansprüche 5 oder 6, dadurch gekennzeichnet, daß das eine Mittel zum Ausführen einer Schwenkbewegung in einer im wesentlichen zu der Hauptebene einer zu prüfenden Tafel senkrechten Ebene schwenkbar gelagert ist, so daß es mit der benachbarten Tafelfläche dicht in Verbindung gebracht werden kann.

8. Gerät nach einem der Ansprüche 5 bis 7, dadurch gekennzeichnet, daß das eine Mittel gleich dem zweiten Mittel ist.

9. Geräte nach einem der Ansprüche 5 bis 8, dadurch gekennzeichnet, daß das Gerät derart angeordnet ist, daß eine Tafel dann geprüft werden kann, wenn diese im wesentlichen horizontal liegt.

10. Gerät nach Anspruch 9, dadurch gekennzeichnet, daß das erste und das zweite Mittel derart angeordnet sind, daß sie an der oberen bzw. der unteren Fläche der zu prüfenden Tafel anliegen.

11. Gerät nach einem der Ansprüche 5 bis 10, dadurch gekennzeichnet, daß das erste Mittel ein paar von einen gegenseitigen Abstand aufweisenden, drehbaren Elementen umfaßt.

## Revendications

1. Procédé de contrôle de la rigidité d'un panneau de bois (6) présentant une relation sensiblement linéaire entre charge et fléchissement lors de l'essai afin d'engendrer une courbe de fléchissement en charge, le procédé comprenant les étapes consistant à :

mettre une surface principale du panneau (6) sensiblement suivant sa largeur en contact avec un élément d'application de charge (15) et solliciter ledit élément (15) pour qu'il applique une première charge sensiblement linéaire et uniforme sur le panneau (6) tandis que l'autre surface principale du panneau est supportée le long de deux lignes espacées (3) situées de part et d'autre d'une ligne d'application de ladite première charge et parallèlement à celle-ci, afin de faire fléchir le panneau (6), ladite charge ayant une grandeur telle qu'elle se situe dans la partie sensiblement linéaire de la courbe de fléchissement en charge relative au panneau (6) ;

déterminer la grandeur de ladite première charge afin d'obtenir une première mesure ;

exercer une sollicitation supplémentaire sur ledit élément d'application de charge (15) afin d'appliquer une charge additionnelle au panneau pour faire fléchir davantage ledit panneau (6) sur une seconde distance, ladite charge additionnelle se situant également sur ladite partie linéaire de ladite courbe de fléchissement en charge ;

déterminer la grandeur de la charge additionnelle et le degré du fléchissement afin d'obtenir une seconde et une troisième mesures ;

utiliser les mesures obtenues afin de calculer une mesure de la rigidité du panneau (6) ;

caractérisé en ce que ledit panneau de bois (6) est un panneau de bois reconstitué et un appareil (1) comprenant un cylindre (4) à double effet, relié à une extrémité à un support (9) et, à l'autre extrémité, audit élément d'application de charge (15) est prévu, de manière à ce que l'extension d'une extrémité du cylindre (4) applique ladite première charge et l'extension de l'autre extrémité du cylindre (4) applique ladite charge additionnelle.

2. Procédé selon la revendication 1, dans lequel ladite sollicitation supplémentaire succède rapidement à l'application de la première charge.

3. Procédé selon la revendication 1 ou la revendication 2, dans lequel ladite première charge est prédéterminée.

4. Procédé selon l'une quelconque des revendications précédentes, dans lequel ladite seconde distance est prédéterminée.

5. Appareil destiné à mettre en oeuvre le procédé des revendications 1 à 4, ledit appareil comprenant:

un premier moyen (3) destiné à porter contre une surface principale du panneau (6) le long de deux lignes parallèles espacées s'étendant sensiblement suivant la largeur du panneau,

un second moyen (15) destiné à porter contre l'autre surface principale du panneau (6) le long d'une ligne s'étendant sensiblement sur la largeur du panneau (6) entre les lignes parallèles espacées susmentionnées ;

un troisième moyen (4) destiné à solliciter l'un desdits premier et second moyens (3, 15) de façon à appliquer une première charge sensiblement linéaire et uniforme sur sensiblement la largeur du panneau (6), ladite première charge étant sélectionnée de façon à se situer sur la partie sensiblement linéaire de la courbe de fléchissement en charge relative au panneau (6), afin de faire fléchir le panneau sur sa largeur sur une première distance ; et

un quatrième moyen (4) destiné à exercer une sollication supplémentaire sur ledit moyen (3, 15) afin d'appliquer au panneau (6) une charge additionnelle qui se situe également sur ladite partie linéaire de ladite courbe de fléchissement en charge, afin de faire fléchir davantage ledit panneau (6) sur une seconde distance ;

caractérisé en ce que lesdits troisième et quatrième moyens (4) comprennent un cylindre (4) à double effet relié, à une extrémité, à un support (9) et, à l'autre extrémité, audit moyen (3, 15), si bien que l'extension d'une extrémité du cylindre (4) applique ladite première charge et l'extension de l'autre extrémité du cylindre (4) applique ladite charge additionnelle.

6. Appareil selon la revendication 5, caractérisé en ce qu'il comprend en outre un cadre de support sur lequel lesdits premier et second moyens et/ou lesdits troisième et quatrième moyens sont montés.

7. Appareil selon la revendication 5 ou la revendication 6, dans lequel ledit moyen est monté de façon pivotante en vue d'un déplacement par pivotement dans un plan généralement perpendiculaire au plan principal d'un panneau faisant l'objet d'un essai, de manière à ce qu'il puisse s'adapter étroitement à la surface de panneau adjacente.

8. Appareil selon l'une quelconque des revendications 5 à 7, dans lequel ledit moyen est ledit second moyen.

9. Appareil selon l'une quelconque des revendications 5 à 8, ledit appareil étant prévu de façon à contrôler un panneau se trouvant dans une position sensiblement horizontale.

10. Appareil selon la revendication 9, dans lequel lesdits premier et second moyens sont prévus de façon à porter contre les surfaces supérieure et inférieure, respectivement, du panneau faisant l'objet de l'essai.

11. Appareil selon l'une quelconque des revendications 5 à 10, dans lequel ledit premier moyen comprend une paire d'éléments espacés rotatifs.

Fig.1.

EP 0 232 573 B1

Fig.2a. Fig.2b. Fig.2c.

Fig. 3.